(19) Europäisches Patentamt
European Patent Office
Office européen des brevets

(11) **EP 3 332 820 A1**

(12) **EUROPEAN PATENT APPLICATION**
published in accordance with Art. 153(4) EPC

(43) Date of publication:
**13.06.2018 Bulletin 2018/24**

(51) Int Cl.:
**A61M 5/152** *(2006.01)*     **A61M 5/315** *(2006.01)*

(21) Application number: **16832261.8**

(86) International application number:
**PCT/CN2016/092121**

(22) Date of filing: **28.07.2016**

(87) International publication number:
**WO 2017/020773 (09.02.2017 Gazette 2017/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **31.07.2015 CN 201510464642**

(71) Applicant: **Chongqing Beka Med, Inc.**
**Xiema Town**
**Beibei District**
**Chongqing 400712 (CN)**

(72) Inventor: **HE, Xiaojun**
**Geleshan Town**
**Shapingba District**
**Chongqing 400036 (CN)**

(74) Representative: **Müller-Boré & Partner**
**Patentanwälte PartG mbB**
**Friedenheimer Brücke 21**
**80639 München (DE)**

(54) **DRUG SYRINGE**

(57)     The disclosure discloses a drug syringe. The drug syringe comprises: a drug container (10) defining a space and a piston (20) arranged in the space. A drug chamber (30) for accommodating a drug may be formed on a side of the piston in the space; and a constant force providing device (40) is arranged on an opposite side of the piston (20) for providing a thrust to the piston (20), pushing the piston (20) to move along an extension direction of the space, and pushing out the drug in the drug chamber (30) by changing a volume of the drug chamber (30).

Fig. 1

EP 3 332 820 A1

**Description**

**TECHNICAL FIELD**

This disclosure relates to a drug syringe, and in particular to a drug syringe having a transversely flowing liquid drug pushing device.

**BACKGROUND**

In the existing treatment regimes, numerous drugs are made into sustained release capsules to achieve the purpose of delaying the drug effect. This method is simple but the dosage cannot be released accurately and constantly, and hence not suitable for treatments which need an accurate dosage release. In certain cases, for the purpose of continued and constant injection of a liquid drug, a patient must be hospitalized for accurate injection through a complex machine, such as a drip device or an electronic pump. Such a method not only occupies large amounts of medical resources, but also binds the patient to a hospital bed, causing the patient not working and living normally.
For a patient in need of chronical and constant drug injection, there is a need for a method that is self-sustained, so that the patient is independent of the supervision by a hospital and a doctor, and the treatment does not impact her normal work or life state.
At present, an existing constantly flowing drug pump, such as an electronically programmed insulin pump, is complex to use, expensive, and has a large size, resulting in inconveniences to patients' life.

**SUMMARY**

An object of the disclosure is to provide a drug syringe that can at least overcome some defects of the prior art.
In a first aspect, the disclosure provides a drug syringe. The drug syringe includes a drug container defining a space and a piston arranged in the space. A drug chamber for accommodating a drug located on a side of the piston in the space, and a power providing device arranged on an opposite side of the piston for providing a thrust to the piston, pushing the piston to move along an extension direction of the space, and pushing out the drug in the drug chamber.
In an embodiment, a spring having a high compression ratio and a spring having a short stroke may be used as the power providing device. In another embodiment, the power providing device may include: a sleeve; and a first spring (small) and a second spring (large) connected adjacently inside the sleeve. The first spring (small) has a smaller cross-section than a cross-section of the second spring (large), so that when the first spring (small) and the second spring (large) are compressed, the compressed first spring is compressed within the compressed second spring (large).
According to the aspect of the embodiment, the power providing device has a small stiffness coefficient and a high compression ratio, to ensure the spring having a very short operating stroke in the working process, thereby achieving the purpose of an approximate constant force.
The drug syringe according to an embodiment of the disclosure may further include a flow rate limiter. The flow rate limiter is arranged in a drug fluid receiving direction of the drug chamber for limiting a flow rate of a liquid drug pushed out from the drug chamber.
In an embodiment, the flow rate limiter has a thin tubular structure to precisely and constantly effect the flow rate of the liquid drug by utilizing a flow rate limiting effect of a capillary. In another embodiment, the flow rate limiter includes a tubule for receiving the liquid drug pushed out from the drug chamber; and a column arranged in the tubule. The column forms a gap with the inner wall of the tubule allowing the received liquid drug to pass through. The length of the column within the tubule is preset to adjust the flow rate of the liquid drug passing through the gap.
In an embodiment, a needle hose is provided in a fluid outflow direction of the flow rate limiter for transporting the liquid drug to a human body by a needle connected to the hose.
In a second aspect, the disclosure further provides a drug syringe system. The system may include the drug syringe as mentioned above; and a base for supporting the drug syringe. The base has a preset opening to enable the needle hose of the drug syringe to pass through. When the drug syringe system is pasted to a patient's skin through a pasting part, a drug enters the patient's body through the needle hose.
The disclosure further simplifies a mechanical constant flow pump/drug syringe to achieve chronical and constant injection of a drug in a simplest mechanical mode. The drug syringe may be attached to a patient's skin by, e.g., pasting using an adhesive plaster, and the drug enters the patient's body through an indwelling hose, so that the patient may be freely reliably and efficiently treated in her normal life or working state.
The technical solution is similar to the existing spring-driven constant flow pump, but does not require a hydraulic part. After the hydraulic part is removed, in order to make up for the precision problem caused by an increased operating stroke of the spring, a free length of the spring is greatly increased using a composite sleeved spring, thus reducing the error between both ends of the operating stroke of the power providing spring.

**BRIEF DESCRIPTION OF THE DRAWINGS**

Fig. 1 is a schematic diagram of a drug syringe according to an embodiment of the disclosure;

Fig. 2 is a structural diagram of a power providing device according to an embodiment of the disclosure;

Fig. 3 is a schematic diagram of a spring for a power providing device according to an embodiment of the disclosure;

Fig. 4 is a plan sketch of a tubular flow rate limiter according to an embodiment of the disclosure; and

Fig. 5 is a schematic diagram of a drug syringe system according to an embodiment of the disclosure.

**DETAILED DESCRIPTION OF EMBODIMENTS**

By referring to the accompanying drawings, the embodiments of the disclosure are further described below. In the accompanying drawings, like reference numbers use like reference symbols, and for the purpose of clear description, some components and parts are omitted in the accompanying drawings.

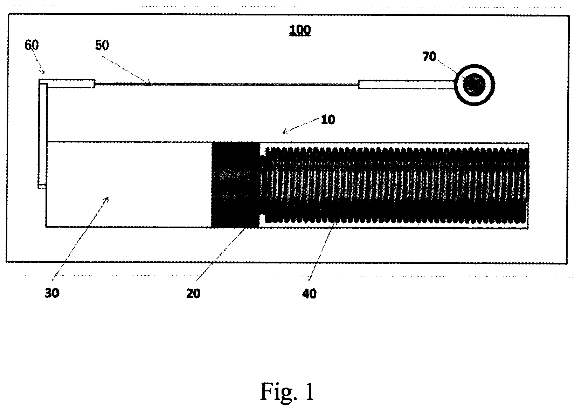

Fig. 1 is a schematic diagram of a drug syringe 100 according to an embodiment of the disclosure. As shown in Fig. 1, the drug syringe 100 includes a drug container 10. The drug container 10 may have, e.g., a tubular space. A piston 20 that may move along an extension direction of the space is arranged in the space. The piston 20 moves along the space to form a drug chamber 30 having a variable volume and for accommodating a drug on a side of the space. On the opposite side of the space, a power providing device 40 is directly fixed to a drug piston. The powder providing device 40 can provide a constant force to the drug piston, pushing the piston 20 to move along an extension direction of the space, and pushing out the drug in the drug chamber 30 by changing a volume of the drug chamber 30. In an embodiment, the power providing device 40 may be a constant force providing device.

In the embodiment, compared to the prior art, because the hydraulic part is removed, the volume of the liquid hydraulic chamber of the drug chamber (hydraulic part) is increased by several times, and now directly pushing the drug piston is equal to 1:1 when the diameter variation ratio is 4:1, since the volume is increased by 4 times. If the diameter of the drug chamber cannot be increased a lot (limited by the device thickness), then the operating stroke of the spring is increased by several times. In the circumstance that the free length of the spring cannot be increased, it will be very difficult to keep a constant force in the process of beginning pushing the spring to completing pushing the spring. If the spring is still considered as a constant force spring in this case, then there will be a very large error of the constant force at the beginning and at the end. Therefore, a constant force providing device must be redesigned to ensure that the device has enough precision.

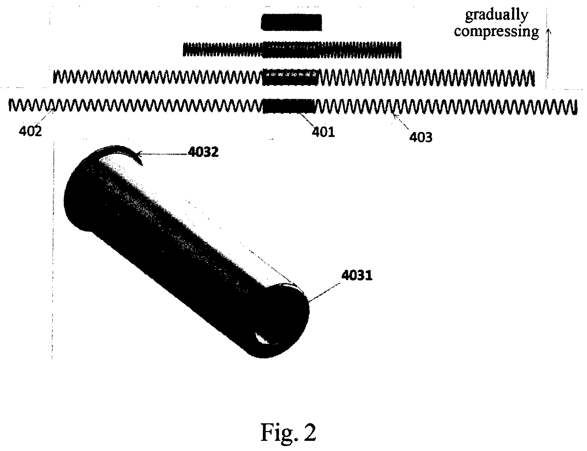

In an embodiment of the disclosure, a power providing device may include a power providing spring having a high compression ratio and a short stroke. The power providing deice may further include a power providing spring having a small stiffness coefficient. Fig. 2 shows a structural diagram of a power providing device 40 according to an embodiment of the disclosure. As shown in Fig. 2, the power providing device 40 includes a sleeve 401; and a first spring 402 (small) and a second spring 403 (large) respectively arranged inside the sleeve 401. The sleeve may be made of metal or plastic, such as ABS. A projection 4031 for limiting the second spring 403 and a projection 4032 for limiting the first spring are provided at an end of the sleeve 401. The first spring 402 and the second spring 403 are connected adjacently, and the first spring 402 (small) has a smaller cross-section than the cross-section of the second spring 403 (large), so that when the two springs are compressed, the compressed first spring 402 (small) is compressed within the compressed second spring 403 (large) . The two springs may be compressed through a push rod (not shown) arranged inside the sleeve. The two springs may be made of an ordinary spring steel or stainless steel, and have an identical stiffness coefficient. Fig. 2 further separately shows a schematic diagram of a configured structure of the sleeve 401 and a state of gradually compressing the first spring 402 (small) and the second spring 403 (large).

Due to the size limitation, in certain cases, the space in the device for the spring is limited. The spring design needs to increase the free length of the spring as much as possible, so the compressed length of the spring is increased accordingly. The design of a spring sleeved inside another spring as shown in the figure is adopted, in which a spring having a larger diameter is in the outside, a spring having a slightly smaller diameter is in the inside, and the two springs are connected end to end with a connecting sleeve to greatly increase the length of the springs under the condition of a given size.

Assume that the spring with a large diameter has a free length of L1 and a compressed length of L0; the spring with a small diameter has a free length of L2 and a compressed length of L0; and a length of the connecting sleeve is approximately equal to L0, a total free length of the assembled spring is L1+L2-L0, wherein the compressed length is L0. Therefore the total compressed length of the spring in the working state is L=L1+ L2-2×L0.

If L1=200MM, L2=150MM and L0=25MM, then L=200+150-50=300MM.

If the spring stroke is $^\triangle$L=20MM,

then the error=ΔL/L=20÷300=0.067=±3.3%.

If a single spring, instead of a composite spring design, is used, then

L=200-25=175 mm.

When the same stroke is ΔL=20MM,

a much larger error=ΔL/L=20÷175=0.114=±5.7% will be obtained.

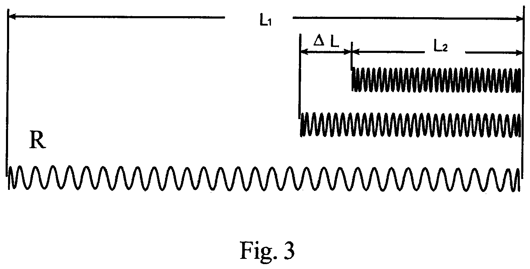

Fig. 3 shows a schematic diagram of a spring adaptable to a power providing device according to an embodiment of the disclosure. The spring R has a small stiffness coefficient and a high compression ratio to facilitate achieving the targeted approximate constant force with a very short operating stroke of the spring R.

As shown in Fig. 3, the free length of the spring R is L1, the length of the spring is compressed to L2 when the spring R works under the action of a drug piston 40, and the working length of the spring is ΔL. When a spring having a stiffness coefficient of k is used, the pressure difference (error) in the actual working process may be calculated using the following formula:

$$\Delta F = F_{max} - F_{min} = k(L_1 - L_2) - k(L_1 - L_2 - \Delta L) = k\Delta L.$$

As can be seen from the formula, the error may be reduced by reducing the stiffness coefficient and shortening the operating stroke ΔL. Therefore, in an embodiment of the invention, the spring having a small stiffness coefficient is used and a short stroke is used to obtain an effect of an approximate constant force spring.

As shown in FIG. 1, a flow rate limiter 50 is arranged in a drug fluid receiving direction of the drug chamber 30 for limiting a flow rate of a liquid drug pushed out from the drug chamber 30. The flow rate limiter 50 is in fluid connection with the drug chamber 30 through a conventional connection part 60. The connection part 60 may be made of, e.g. , a plastic material suitable for a drug, such as polypropylene.

In an embodiment, the flow rate limiter 50 may have a capillary structure to precisely and constantly effect the flow rate by utilizing a flow rate limiting effect of a capillary, i.e., compensating for an inner diameter error using a length. When a fluid passes through a tubule having a round cross-section very slowly, such a flow is laminar flow. The flow rate of the fluid in the round tube is associated with the pressure difference at both ends of the tube, the viscosity of the fluid per se, the length of the tube and the diameter of the round tube. When it is used as a flow rate limiter, the exact flow rate may be calculated by the following equation:

Q=0.014625×(D4×ΔP)/(η×L).

Q: flow rate (μL/day)

ΔP: pressure drop on the flow rate limiter (psi)

η: viscosity of a liquid passing through the flow rate limiter 50 (cp)

L: length of the round tube of the flow rate limiter 50

D: Radius of the round tube (μm)

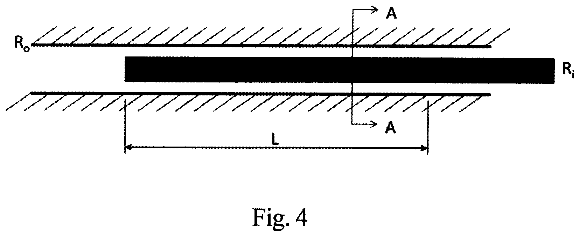

In another embodiment, the flow rate limiter 50 may include a tubule 501 and a column (such as metal wire) 502 inserted into the capillary 501. A gap allowing a liquid drug to circulate is formed between the tubule 501 and the inserted column 502. For example, the tubule 501 may be, for instance, a round tube, the column 502 may be, for instance, a cylinder, and then the round tube and the inserted cylinder form a tubular flow rate limiter of an annular flow. Fig. 4 is a plan sketch of a tubular flow rate limiter.

As shown in FIG. 4, the flow rate limiter 50 adjusts the flow rate of the flow rate limiter through a stack length L of the round tube and the cylinder. It should be understood that the stack length L may be preset based on specific needs. When the flow rate is slow (laminar flow), the liquid flow rule in the annular flow tube is:

Q=(Π ΔP)/8ηL[ Ro4-Ri4-(Ro2-Ri2)2/Ln(Ro/Ri)].

Q: Flow rate

Δp: Pressure drop on the flow rate limiter

H: Viscosity of the liquid flowing through the flow rate limiter

L: Length of the flow rate limiter: stack length of the round tube and the cylinder

Ro: Radius of the round tube

Ri: Radius of the cylinder

Ln: logarithm of a natural number to base e

Further returning to Fig. 1, an indwelling needle hose 70 may also be provided in the fluid outflow direction of the flow rate limiter 50 for transporting the liquid drug to a human body by a needle connected to the interface. The needle hose 70 may be in fluid connection with the flow rate limiter 50 by conventional connection means.

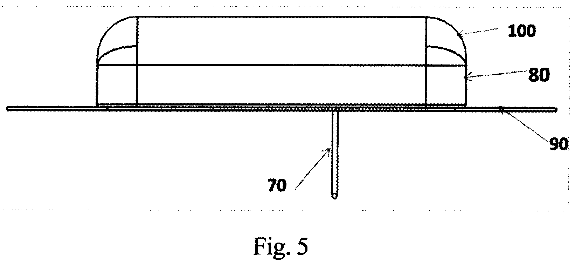

According to an embodiment of the disclosure, as shown in Fig. 5, a drug syringe 100 may be arranged on a base 80, which has a preset opening to enable the needle hose 70 to pass through. A pasting part 90, e.g., an adhesive plaster, may be arranged at the bottom of the base 80 to paste the drug syringe 100 to a patient's skin, and a drug enters the patient's body through an indwelling hose 70, so that the patient may be freely reliably and efficiently treated in her normal life or working state.

A drug syringe and a drug syringe system according to the embodiments of the disclosure are described above. However, it should be understood that the above description only provides embodiments to implement the invention, other than to limit the invention. Any modification, equivalent replacement, improvement and the like of the invention within the spirit and principle of the invention shall be included in the scope of protection of the invention.

**Claims**

1. A drug syringe comprising:

   a drug container (10) defining a space,
   a piston (20) arranged in the space,
   a drug chamber (30) for accommodating a drug located on a side of the piston in the space; and
   a power providing device (40) arranged on an opposite side of the piston (20) for providing a thrust to the piston (20), pushing the piston (20) to move along an extension direction of the space, and pushing out the drug in the drug chamber (30) by changing a volume of the drug chamber (30).

2. The drug syringe according to claim 1, wherein the power providing device comprises a power providing spring providing the thrust.

3. The drug syringe according to claim 1, wherein the power providing device (40) comprises:

   a sleeve (401); and
   a first spring (402) and a second spring (403) connected adjacently inside the sleeve (401),
   wherein the first spring (402) has a smaller cross-section than a cross-section of the second spring (403), so that when the first spring and the second spring are compressed, the compressed first spring is compressed within the compressed second spring.

4. The syringe according to claim 2, wherein the power providing spring has a high compression ratio and a small stiffness coefficient.

5. The drug syringe according to claim 3, wherein the first spring (402) and the second spring (403) have an identical stiffness coefficient.

6. The drug syringe according to any one of claims 1 to 5, further comprising:
   a flow rate limiter (50), arranged in a drug fluid receiving direction of the drug chamber (30) for limiting a flow rate of a liquid drug pushed out from the drug chamber (30) .

7. The drug syringe according to claim 6, wherein the flow rate limiter has a thin tubular structure, to precisely and constantly effect the flow rate of the liquid drug by utilizing a flow rate limiting effect of a capillary.

8. The drug syringe according to claim 6, wherein the flow rate limiter comprises:

   a tubule, for receiving the liquid drug pushed out from the drug chamber (30); and
   a column, arranged in the tubule to form a gap with the inner wall of the tubule allowing the received liquid drug to pass through.

9. The drug syringe according to claim 8, wherein the length of the column within the tubule is preset, to adjust the flow rate of the liquid drug passing through the gap.

10. The drug syringe according to claim 8, wherein a needle hose (70) is provided in a fluid outflow direction of the flow rate limiter (50), for transporting the liquid drug to a human body by a needle connected to the hose.

11. A drug syringe system, comprising:

   the drug syringe according to any one of claims 1 to 10; and
   a base (80), for supporting the drug syringe, and having a preset opening to enable the needle hose (70) of the drug syringe to pass through.

Fig. 1

gradually
compressing

402          401       403

4032

4031

Fig. 2

Fig. 3

Fig. 4

Fig. 5

## INTERNATIONAL SEARCH REPORT

| International application No. |
| --- |
| PCT/CN2016/092121 |

### A. CLASSIFICATION OF SUBJECT MATTER

A61M 5/152 (2006.01) i; A61M 5/315 (2006.01) i

According to International Patent Classification (IPC) or to both national classification and IPC

### B. FIELDS SEARCHED

Minimum documentation searched (classification system followed by classification symbols)

A61M 5

Documentation searched other than minimum documentation to the extent that such documents are included in the fields searched

Electronic data base consulted during the international search (name of data base and, where practicable, search terms used)

CNABS, CNTXT, CNKI, VEN: inject+, transfus+, delivery, piston, push+, urg+, driv+, spring, limit+, flow+, capillary, tube, constant

### C. DOCUMENTS CONSIDERED TO BE RELEVANT

| Category* | Citation of document, with indication, where appropriate, of the relevant passages | Relevant to claim No. |
| --- | --- | --- |
| X | US 5858001 A (ELAN MEDICAL TECHNOLOGIES LIMITED) 12 January 1999 (12.01.1999) description, column 8, line 16 to column 13, line 64 and figures 1-3, 16-20 | 1, 2, 4, 11 |
| Y | US 5858001 A (ELAN MEDICAL TECHNOLOGIES LIMITED) 12 January 1999 (12.01.1999) description, column 8, line 16 to column 13, line 64 and figures 1-3, 16-20 | 6-11 |
| Y | CN 2481337 Y (JIANG, Wenjun) 13 March 2002 (13.03.2002) description, page 3, paragraph 5 to page 4, paragraph 2 and figures 1 and 2 | 6-11 |
| X | CN 102971027 A (BECTON DICKINSON CO.) 13 March 2013 (13.03.2013) description, paragraphs [0021] to [0054] and figures 1-33 | 1, 2, 4, 11 |

☒ Further documents are listed in the continuation of Box C.        ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 October 2016 | 27 October 2016 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>WEI, Na<br><br>Telephone No. (86-10) 62089912 |

Form PCT/ISA /210 (second sheet) (July 2009)

<table>
<tr><td colspan="2" align="center"><strong>INTERNATIONAL SEARCH REPORT</strong></td><td>International application No.<br>PCT/CN2016/092121</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| X | US 2009088724 A1 (TYCO HEALTHCARE GROUP LP) 02 April 2009 (02.04.2009) paragraphs [0030] to [0047] and figures 1-4 | 1,2,4 |
| X | CN 104245012 A (SANOFI-AVENTIS DEUT GMBH) 24 December 2014 (24.12.2014) paragraphs [0115] to [0127] and figures 1-5 | 1,2,4 |
| X | WO 2014146210 A1 (TECPHARMA LICENSING AG) 25 September 2014 (25.09.2014) description, page 22, line 11 to page 30, line 29 and figures 1-5 | 1,2,4 |
| A | CN 201164626 Y (HAO, Tianyu  et al.) 17 December 2008 (17.12.2008) the whole document | 1-11 |

Form PCT/ISA/210 (continuation of second sheet) (July 2009)

INTERNATIONAL SEARCH REPORT
Information on patent family members

International application No.

PCT/CN2016/092121

| Patent Documents referred in the Report | Publication Date | Patent Family | Publication Date |
|---|---|---|---|
| US 5858001 A | 12 January 1999 | EP 0902696 B1 | 27 March 2002 |
| | | DE 69620257 D1 | 02 May 2002 |
| | | AT 214954 T | 15 April 2002 |
| | | AU 1808797 A | 03 July 1997 |
| | | TW 317503 B | 11 October 1997 |
| | | IL 124470 D0 | 06 December 1998 |
| | | DE 69620257 T2 | 07 November 2002 |
| | | EP 0902696 A1 | 24 March 1999 |
| | | ZA 9610374 A | 23 June 1997 |
| | | JP 2000515394 A | 21 November 2000 |
| | | ZA 9610374 B | 23 June 1997 |
| | | CA 2238614 A1 | 19 June 1997 |
| | | WO 9721457 A1 | 19 June 1997 |
| CN 2481337 Y | 13 March 2002 | None | |
| CN 102971027 A | 13 March 2013 | AU 2011256804 A1 | 10 January 2013 |
| | | SG 185653 A1 | 28 December 2012 |
| | | JP 2016073722 A | 12 May 2016 |
| | | ES 2571331 T3 | 24 May 2016 |
| | | CA 2799721 A1 | 24 November 2011 |
| | | EP 2571549 B1 | 24 February 2016 |
| | | CN 104984438 A | 21 October 2015 |
| | | WO 2011146166 A1 | 24 November 2011 |
| | | AU 2011256804 B2 | 17 October 2013 |
| | | MX 2012013426 A | 12 February 2013 |
| | | CA 2935653 A1 | 24 November 2011 |
| | | SG 10201503939 SA | 30 July 2015 |
| | | US 2013110049 A1 | 02 May 2013 |
| | | CN 102971027 B | 26 August 2015 |

Form PCT/ISA /210 (patent family annex) (July 2009)

## INTERNATIONAL SEARCH REPORT
### Information on patent family members

International application No.

PCT/CN2016/092121

| Patent Documents referred in the Report | Publication Date | Patent Family | Publication Date |
|---|---|---|---|
| | | US 9408985 B2 | 09 August 2016 |
| | | EP 3028726 A1 | 08 June 2016 |
| | | JP 5934188 B2 | 15 June 2016 |
| | | EP 2571549 A1 | 27 March 2013 |
| | | JP 2013526366 A | 24 June 2013 |
| | | IN 201210158 P1 | 05 September 2014 |
| US 2009088724 A1 | 02 April 2009 | EP 2042210 A3 | 28 July 2010 |
| | | EP 2042210 A2 | 01 April 2009 |
| | | JP 2009082715 A | 23 April 2009 |
| | | MX 2008012214 A | 15 April 2009 |
| | | IL 194286 D0 | 03 August 2009 |
| | | CA 2639729 A1 | 27 March 2009 |
| | | MX 306216 B | 17 December 2012 |
| CN 104245012 A | 24 December 2014 | WO 2013124281 A1 | 29 August 2013 |
| | | EP 2817039 A1 | 31 December 2014 |
| | | HK 1199627 A1 | 10 July 2015 |
| | | JP 2015511157 A | 16 April 2015 |
| | | US 2015018779 A1 | 15 January 2015 |
| WO 2014146210 A1 | 25 September 2014 | US 2016008542 A1 | 14 January 2016 |
| | | JP 2016512766 A | 09 May 2016 |
| | | US 2016008541 A1 | 14 January 2016 |
| | | EP 2742962 A3 | 23 July 2014 |
| | | EP 2742962 A2 | 18 June 2014 |
| | | EP 2781230 A1 | 24 September 2014 |
| | | JP 2016512767 A | 09 May 2016 |
| | | CN 105246528 A | 13 January 2016 |
| | | WO 2014146209 A1 | 25 September 2014 |
| CN 201164626 Y | 17 December 2008 | None | |

Form PCT/ISA/210 (patent family annex) (July 2009)